# EUROPEAN PATENT APPLICATION

(11) **EP 1 616 941 A1**
(43) Date of publication of application: **18.01.2006**
(21) Application number: 04722404.3
(22) Date of filing: 22.03.2004
(51) Int. Cl.: C12N 5/00, A61F 2/14, A61L 27/00

(54) **METHOD OF PRODUCING LENS CELL AND LENS CELL OBTAINED BY THE METHOD**

(30) Priority: 31.03.2003 JP 2003096002
(71) Applicant: Japan Science and Technology Agency, Kawaguchi-shi, Saitama 332-0012 (JP)
(72) Inventor: TAKAHASHI, Masayo, Kyoto-shi, Kyoto 606-8336 (JP); SASAI, Yoshiki, Kobe-shi, Hyogo 657-0066 (JP); KAWASAKI, Hiroshi, Kanazawa-shi, Ishikawa 920-0831 (JP); OOTO, Sotaro, Kyoto-shi, Kyoto 602-8241 (JP)
(74) Representative: Müller - Hoffmann & Partner
(86) International application number: PCT/JP2004/003848
(87) International publication number: WO 2004/087897

(57) **Abstract**

A method of producing a lens cell according to the present invention includes: an ES cell maintenance step of maintaining an ES cell by using a medium containing a fibroblast growth factor FGF-2 at a concentration of 2 ng/ml to 50 ng/ml; and a differentiation inducing step, carried out after the ES cell maintenance step, of inducing differentiation of the ES cell into a lens cell by implanting and culturing the ES cell on a mouse-skull-cell PA6 at a cell density of 2 colonies/cm² to 6.5 colonies/cm². It is preferable that a washing step, carried out between the ES cell maintenance step and the differentiation inducing step, of washing the maintained ES cell once with an ES differentiation medium be further included.

## Description

### TECHNICAL FIELD

The present invention relates to a novel method of producing lens cells, applicable to treatment of cataract and other eye diseases, whereby differentiation of embryonic stem (ES) cells into lens cells is induced, and to lens cells obtained by using the method.

### BACKGROUND ART

Cataract is an eye disease that causes a clouding of the lens due to a variety of causes. Since the clouded lens leads to vision problems, cataract is a serious eye disease. Currently, cataract is commonly treated by surgery in which a clouded lens is removed from a lens capsule, and an artificial intraocular lens is fixed in the capsular bag. For the treatment using intraocular lenses, there have been ongoing development of softer intraocular lenses which allow for a smaller incision of the eyeball.

However, since the artificial intraocular lenses cannot adjust the lens thickness, the adjusting ability of natural lenses cannot be attained with the artificial lenses. Thus, the cataract surgery in which intraocular lenses are transplanted cannot improve presbyopia due to aging, which occurs when the ability of the lens to focus on a nearby object weakens due to reduced elasticity of the lens. In addition, in cataracts among young people, surgery is often not recommended because the surgery instantly leads to presbyopia.

Such problems can be solved by culturing human lens cells using the recently advanced tissue-culture technology and using the cultured cells as biomaterials for transplant. To this end, there has been actively research. For instance, Patent Document 1 (United States Patent No. 5,643,782) discloses a method of producing immortalized human lens epithelial cells by infecting human lens epithelial cells with hybrid adenovirus SV40. Further, Patent Document 2 (United States Patent No. 5,885,832) discloses a method of producing immortalized human lens epithelial cells by transfecting human lens epithelial cells with immortalized genes (genes coding for SV40 large T antigen).

However, immortalization of cells as described in Patent Documents 1 and 2 always has the risk of cells growing into tumors. Further, the infection by adenovirus has the risk of causing inflammation.

As described above, although there has been active research on the technique of culturing lens cells to be used as biomaterials, there has been no established method of producing human lens cells practically used as biomaterials.

Meanwhile, recently, embryonic stem cells (ES cells) of primates or humans have been isolated, and regenerative treatment is drawing attention as future medicine (see Patent Document 3: United States Patent No. 5,843,780, Patent Document 4: United States Patent No. 6,200,806). Once the method of inducing ES cells into lens cells is established by using the regenerative treatment, then it will be possible to reproduce lenses having an adjusting ability by implanting the lens cells into the vescicle. This makes it possible to treat cataract as well as presbyopia due to aging. In addition, since the production of such ES cell-derived lens cells do not involve manipulation of cells as described in Patent Documents 1 and 2, the lens cells are closer to the natural lens cells, offering greater applicability to biomaterials.

Non-Patent Document 1 (Kawasaki H., Mizuseki K., Nishikawa S. et. al., Induction of midbrain dopaminergic from ES cells by stromal cell-derived inducing activity, Neuron, 2000, Vol. 28, pp. 31-40) and Non-Patent Document 2 (Kawasaki H., Suemori H., Mizuseki K. et. al., Generation of dopaminergic neurons and pigmented epithelia from primate ES cells by stromal cell-derived inducing activity, Proc Natl Acad Sci USA, 2002, Vol. 99 pp. 1580-1585) report that nerve cells and retinal pigment epithelial cells can be produced by applying the SDIA method as a method of inducing differentiation of ES cells. However, the methods described in the above publications cannot produce a group of homogeneous cells.

As described above, reproduction of lenses for practical application has not been successful in primates, and establishment of a reproducing method is expected. The present invention provides a method for stably producing lens cells by inducing differentiation of ES cells, and lens cells produced by the method.

### DISCLOSURE OF INVENTION

After having diligently studied the problems described above, the inventors of the present application found that lens cells can be stably produced by applying the SDIA (Stromal-Cell-Derived Inducing Activity) method described in Non-Patent Documents 1 and 2 and by modifying conditions for maintaining ES cells and inducing differentiation, and have completed the present invention.

A method of producing a lens cell according to the present invention includes: an ES cell maintenance step of maintaining an ES cell by using a medium containing a fibroblast growth factor FGF-2 at a concentration of 2 ng/ml to 50 ng/ml; and a differentiation inducing step, carried out after the ES cell maintenance step, of inducing differentiation of the ES cell into a lens cell by implanting and culturing the ES cell on a mouse-skull-cell PA6 at a cell density of 2 colonies/cm² to 6.5 colonies/cm².

The above method allows a lens cell to be stably produced from ES cells, and therefore provides lens cells that can be used as biomaterials for transplant, and lens fiber tissues formed of such lens cells. The lens cells and their tissues are also referred to as lentoids in this description. Using lens cells and tissues (lentoids) produced by the above method as biomaterials for transplant allows lenses to have the adjusting ability of lens thickness that is similar to the adjusting ability of the natural lenses, in contrast to the artificial intraocular lenses used in the conventional treatment for cataracts.

Further, the lens cells derived from ES cells are closer to natural lens cells, compared to the lens cells obtained by manipulating cells as described in above Patent Documents 1 and 2. Therefore, the lens cells derived from ES cells offer greater applicability to biomaterials. In addition, the lens cells obtained by the above method shed light on various mechanisms relating to induction of differentiation using ES cells, are expected to contribute to development in pharmaceutical field, and are highly useful.

Further, it is preferable in a method of producing a lens cell in the present invention that a washing step, carried out between the ES cell maintenance step and the differentiation inducing step, of washing the maintained ES cell once with an ES differentiation medium be further included.

In the above method, differentiation of ES cells into lens cells is induced in a favorable manner, and a group of homogeneous lens cells can be obtained. Therefore, the lens cells obtained by the above method may be used as biomaterials, thereby contributing to development of regenerative treatment.

Further, it is preferable in a method of producing a lens cell in the present invention that the ES cell be derived from primates.

In the above method, because humans belong to primates, lens cells that are similar to human lens cells can easily be produced in a large quantity. Therefore, the lens cells produced by the above method are useful in research and development of various therapeutic drugs for various eye diseases in humans. The method of producing a lens cell in the present invention is the first case of successful induction of differentiation of ES cells of humans and other primates into lens cells.

Concrete examples of the primates include human, chimpanzee, gorilla, pongo, and cynomolgus monkey. In the example below, with respect to ES cells of cynomolgus monkey, lens cells are actually induced to differentiate by a method of the present invention. From the example, it is confirmed that the ES cells of cynomolgus monkey go through a differentiation process similar to the differentiation process that primate lens cells in vivo go through. Therefore, it is preferable that a method of producing a lens in the present invention use cynomolgus monkey-derived ES cells.

Further, lens cells in the present invention are obtained by the above method.

Because the lens cells constitute a group of homogeneous cells reproduced from the ES cells, the lens cells can be effectively utilized for development of biomaterials for transplant in regenerative treatment. A concrete example of methods in which a lens in the present invention may be used is a method in which lens cells are implanted in a lens capsule, and in which soft lenses whose lens thickness is adjustable are reproduced. This method is useful for treatment of cataracts, and has a merit that cataracts and presbyopia are treated at the same time.

Further aims, features, and advantages of the present invention will be fully understandable from the description below. In addition, the merits of the present invention will also be clear from the following description.

### BRIEF DESCRIPTION OF DRAWINGS

Figures 1(a) to 1(d) show results of phase contrast microscopy on lentoids that were induced to differentiate in an Example of the present invention.
Figure 2 shows a result of immunostaining performed on lentoids that were induced to differentiate in an Example of the present invention.
Figures 3(a) and 3(b) are diagrams showing results of western-blotting analysis performed to investigate protein expression in the cells that were induced to differentiate in one Example of the present invention, in which Figure 3(a) shows a result of investigation on expression of alpha-crystallin A, and Figure 3(b) shows a result of investigation on expression of Pax6.
Figure 4 is a graph showing a relationship between elapsed time from the start of induction and percent induction of lentoids, at different FGF-2 concentrations of 2 ng/ml, 4 ng/ml, and 8 ng/ml in the medium for maintaining undifferentiated ES cells.
Figure 5 is a graph showing a relationship between elapsed time from the start of induction and percent induction of lentoids, at high and low ES cell densities.
Figure 6 is a graph showing percent induction of lentoids at different FGF-2 concentrations thirty days after the start of induction, at high and low ES cell densities.
Figure 7(a) is a diagram showing a result of microscopy of lentoids cultured at a high colony density. Figure 7(b) is a diagram showing a result of microscopy of retinal pigment epithelial cells cultured at a high colony density.

### BEST MODE FOR CARRYING OUT THE INVENTION

The following describes the present invention in detail, but the present invention is not limited by the description.

A method of producing a lens cell in the present invention includes an ES cell maintenance step of maintaining an ES cell by using a medium containing a fibroblast growth factor FGF-2 at a concentration of 2 ng/ml to 50 ng/ml; and a differentiation inducing step, carried out after the ES cell maintenance step, of inducing differentiation of the ES cell into a lens cell by implanting and culturing the ES cell on mouse-skull-bone-cells PA6 at a cell-density of 2 colonies/cm² to 6.5 colonies/cm².

The method of producing a lens cell employs the SDIA method in which mouse-skull-bone-marrow cells PA 6 are used as feeder-cells for the induction of differentiation of ES cells. The method is applicable to the ES cells of primates, particularly humans. Primary features of the method of producing a lens cell according to the present invention reside in:
(1) The final concentration of FGF-2 added to a medium used for maintaining undifferented ES cells;
(2) The cell-density of implanted ES cells on PA6; and
(3) The number of times the ES cells are washed before the ES cells are implanted on PA6.

The following describes the above three features in this order. Initially, description is made as to (1) the final concentration of FGF-2 added to a medium used for maintaining undifferented ES cells.

In a method of producing a lens cell according to the present invention, ES cells in the ES cell maintenance step are maintained by using a medium containing a fibroblast growth factor FGF-2 at a concentration of between 2 ng/ml and 50 ng/ml. As used herein, "maintaining ES cells" means maintaining ES cells in an undifferentiated state, and the "ES cell maintenance step" includes suitably sub-culturing ES cells. Further, the medium includes various types of mediums that are capable of culturing and maintaining cells.

In the ES cell maintenance step, the final concentration of the fibroblast growth factor FGF-2 in the medium is set to 2 ng/ml or greater, so as to induce differentiation of lentoids, as will be described in the Example below. Further, in the ES cell maintenance step, the final concentration of FGF-2 is set to 50 ng/ml or less, because, if the final concentration of FGF-2 in the medium is too high, cell grows so rapidly that the maintenance becomes difficult.

Further, as will be described in the Example below, induction of differentiation of lentoids is more facilitated in a FGF-2 concentration of 4 ng/ml than in 2 ng/ml. The induction of differentiation of lentoids is even more facilitated in 8 ng/ml than in 4 ng/ml. Therefore, it is preferable that the concentration of the medium maintaining FGF-2 in the ES cell maintenance step be between 4 ng/ml and 5 ng/ml, or more preferably between 8 ng/ml and 32 ng/ml for more desirable induction of differentiation.

As to conditions other than the concentration of FGF-2 in the ES cell maintenance step, those employed in conventional methods can be used. Concrete examples of the maintenance method are described in the Example.

The following describes the second feature of the present invention, i.e., (2) the cell density of implanted ES cells on PA6.

Normally, in the induction of ES cells differentiation by the SDIA method, the differentiation of the ES cells is induced by implanting the ES cells after the ES cell maintenance step, using mouse-skull-bone-marrow cells PA6 as feeder-cells. In a method of producing a lens cell according to the present invention, in the differentiation inducing step, the ES cells are implanted on feeder-cells PA6 in an undifferentiated state at the cell density of between 2 and 6.5 (colonies/cm²). The cell density of 2 to 6.5 (colonies/cm²) is equivalent to about 150 to 500 colonies when converted into the number of colonies per culture dish, 10 cm in inner diameter, that contains the PA6-cells. In other words, in a method of producing a lens cell according to the present invention, the cell density of implanted ES cells on PA6-cells in the differentiation inducing step is between 150 and 500 (colonies/culture dish with an inner diameter of 10 cm).

As described above, with the cell density of implanted ES cells on PA6 set to 2 (colonies/cm²) or greater, the ES cells of especially, primates can be induced to differentiate into lens cells, as will be described in the Example below. Further, as in the case of FGF-2 concentration, maintenance of the cells becomes difficult when the ES cell density is too high. Therefore, in the present invention, the upper limit of ES cell density is set to 6.5 (colonies/cm²) or less.

Further, in a method of producing a lens cell according to the present invention, it is preferable that the cell density of implanted ES cells on PA6-cells be between 2.5 and 4.0 (colonies/cm²). The cell density of 2.5 to 4.0 (colonies/cm²) is equivalent to about 200 to 300 colonies when converted into the number of colonies per culture dish, 10 cm in inner diameter, that contains PA6-cells. In other words, in the method of producing a lens cell according to the present invention, it is preferable that the cell density of implanted ES cells on the PA 6 cells in the differentiation inducing step be between 200 and 300 (colonies/culture dish with an inner diameter of 10 cm). The cell density of 200 to 300 (colonies/culture dish with an inner diameter of 10 cm) is approximately 1.5 to 2 times greater than the cell density for inducing neurons. Carrying out the implantation at the above cell-density makes it possible to stably supply a group of homogeneous lens cells.

In the differentiation inducing step in a method of producing a lens cell according to the present invention, the ES cells implanted on PA6 can be cultured under the culture conditions conventionally employed for the differentiation induction of the ES cells. Specifically, it is preferable that the culture-temperature be between 34°C and 38°C, and the culture-pH be between pH 6.0 and pH 8.0. With regard to the culture system, a suitable system can be selected from a static culture, rotary culture, and penetration culture, for example.

The following describes another feature of the present invention, i.e., (3) the number of times the ES cells are washed before the ES cells are implanted on PA6.

In a method of producing a lens cell according to the present invention, the ES cells are washed between the ES cell maintenance step and the differentiation inducting step. In the present invention, it is preferable that the undifferentiated ES cells maintained in the ES cell maintenance step be washed only once with an ES differentiated medium to wash off the maintenance medium.

In a conventional SDIA method, undifferented ES cells are washed three times, as opposed to once in the present invention. By washing the ES cells only once, the differentiation of the ES cells into the lens cells can be induced more desirably, and a group of homogeneous lens cells can be obtained.

The ES differentiation medium used for the washing of the ES cells is preferably the differentiation inducing medium used for inducing differentiation of the ES cells into the lens cells. In addition, it is preferable that the ES differentiation medium be used in an amount 30 to 100 times greater than the volume of an undifferentiated ES cell pellet.

By inducing differentiation of the ES cells into the lens cells according to the foregoing method of producing a lens cell, the lens cells and tissues (lentoids) can be stably obtained after 2 to 3 weeks of incubation on the PA6 cells.

As will be described in the Example below, differentiation can be induced more effectively when the FGF-2 concentration in the ES cell maintenance medium and the cell density of implanted ES cells are increased together. Therefore, by increasing both the FGF-2 concentration and the ES cell density within the range described above, lens cells can be stably produced with improved quality.

It is preferable that a method of producing a lens cell according to the present invention be carried out with the ES cells of primates including humans. With the invention applied to the ES cells of primates, lens cells that are similar to the human lens can easily be produced in a large quantity. This is beneficial in the research and development of therapeutic drugs for various eye diseases in humans.

Further, lens cells of the present invention are produced by a method of producing a lens cell according to the present invention, and therefore the present invention includes a variety of lens cells produced under suitably modified conditions within a scope of the present invention.

The lens cells of the present invention are useful for finding mechanisms of ES cell differentiation, and for the research and development of therapeutic drugs for cataracts. Moreover, the lens cells of the present invention have potential use as an intraocular lens with superior adjustability of lens thickness compared with an artificial intraocular lens.

### [Example]

The following describes one Example of the present invention, but the present invention is not limited by the description. For the induction of differentiation into lens cells, ES cells of cynomolgus monkey were used herein as an example of primate ES cells.

### [1] Methods of Experiment

The Example was carried out through methods (1) to (4) described below.

### (1) Maintaining ES Cells

First of all, the following describes how ES cells of cynomolgus monkey used in the experiment were obtained.

ES cell lines were obtained from the blastocyst of cynomolgus monkey, in accordance with the procedure described in Suemori H., Tada T., Torii R. et. al., Establishment of embryonic stem cell lines from cynomolgus monkey blastocyst produced by IVF or ICSI, Dev Dyn., 2002, Vol. 222, pp. 273-279. Pluripotency of the ES cell line was confirmed.

Undifferentiated ES cells were placed on a feeder-layer of mouse embryonic fibroblast (STO cells) inactivated with mitomycin C. The cells were cultured in a DMEM/F-12 medium (Sigma) supplied with 0.1 mM 2-mercaptoethanol (Sigma), 1000 U/ml leukemia inhibitory factor (ESGRO, CHEMI-CON), 20% knockout serum alternative (Gibco), 0.1 mM non-essential amino acid (Gibco), and 8 ng/ml fibroblast growth factor (FGF-2, Upstate). The medium was replaced everyday.

Subsequently, subculture of the ES cells was treated with a 1 mM CaCl₂PBS solution containing 0.25% trypsin, and a 20% knockout serum alternative. From three to four days before implantation of the ES cells on the mouse-skull-bone-marrow-derived PA6, the fibroblast growth factor FGF-2 was added to culture media at concentrations of 2 ng/ml, 4 ng/ml, and 8 ng/ml, respectively.

### (2) Induction of Lentoid

Subsequently, the ES cells maintained on the media containing different concentrations of FGF-2 were induced to differentiate through the procedures below.

PA6 was implanted in a gelatin-coated culture dish so as to be used as a feeder-cell layer. After trypsinization, a group of partially separated ES cells (30-50 cells/one group) were inoculated on a GMEM medium (Gibco) containing a gelatin-coated 10% FBS (Hyclone). After 30 minute incubation at 37°C, the ES cells were dispersed by pipeting. Cell pellets collected by centrifugation were washed with the ES differentiation medium [10% knockout serum alternative, 1mM pyruvic acid (Sigma), 0.1 mM non-essential amino acid, 0.1 mM 2-mercaptoethanol (Wako)].

Subsequently, the washed ES cells were implanted on the PA6 feeder-cell layer to induce differentiation. In this implantation step, the ES cells were implanted on the PA6 cells at two different densities: one with 200 or greater colonies per culture dish with an inner diameter of 10 cm (high density); and one with 150 or greater colonies per culture dish with an inner diameter of 10 cm (low density). The implanted ES cells were cultured in the differentiation medium for at least six weeks. The medium was replaced every three days.

### (3) An Immunohistochemical Examination of Cultured Cells

An immunohistochemical examination was carried out on the cells cultured with the method described in section (2) above.

The cells were kept in 4% paraformaldehyde (Wako) for one hour, and then immersed in 25% sucrose PBS. After washed with 0.1 M phosphate buffer (PB), the sample was incubated with a skimmed milk powder in 0.1 M PB containing 0.005% saponin (0.1 mM PB-saponin, Merck) for one hour to block nonspecific antibody binding. Together with a primary antibody diluted with 0.1 M PB-saponin containing 5% skimmed milk powder, the sample was incubated at 4°C for 24 hours. As the primary antibody, rabbit polyclonal anti-alpha-crystallin A (1:1000, Stressgen) was used. Activity of the antibody was confirmed by using a rat lens as a positive control. After washed with 0.1 M PB, the sample was incubated at room temperature for one hour with a fluorescein-conjugated-burro-anti-rabbit-globulin (1:100, Amersham) secondary antibody diluted with 0.1 M PB-saponin containing 5% skimmed milk powder. After washed with 0.1 M PB, the sample was fixed on a slide with glycerol-PBS (1:1), and was observed under a laser automatic read-in confocal microscope (Leica).

### (4) SDS Polyacrylamido Gel Electrophoresis and Western-Blotting Analysis

SDS polyacrylamido gel electrophoresis and western-blotting analysis were carried out to the cells cultured by the method described in section (2) above.

The cells were collected by scraping and were dissolved in 500 µl of fusion buffer (Laemmli sample buffer, BIO-RAD) containing 5mM 2-mercaptoethanol (Wako). The cell suspension was homogenized on ice. The homogenized cells were stored at -80°C. The ES cell lysate was electrophoresed and the isolated protein was transferred onto a PVDF membrane (Immobilom-P, Millipore). Non-specific antibody binding was blocked by one-hour incubation with a 0.1 M PB containing 20% skimmed milk powder. Blots were incubated at room temperature together with primary antibodies diluted with 0.1 M PB containing 5% skimmed milk powder. Rabbit polyclonal antibody, anti-alpha-crystallin A (1:1000, Stressgen), and anti-Pax 6 (1:200, Santa Cruz) were used as primary antibodies. A primary antibody binding was detected by using, in accordance with the ABC method, anti-rabbit IgG bound to alkaline phosphatase (1:100, made by Vector Laboratories). After washed with 0.1 M PB, the blots were developed with a phosphatase substrate (KONICA immunostaining, KONICA) in accordance with a protocol.

### [2] Results

The following (1) to (4) describes results of the experiment carried out by using the above methods.

### (1) With Regard to Induction of Lentoids

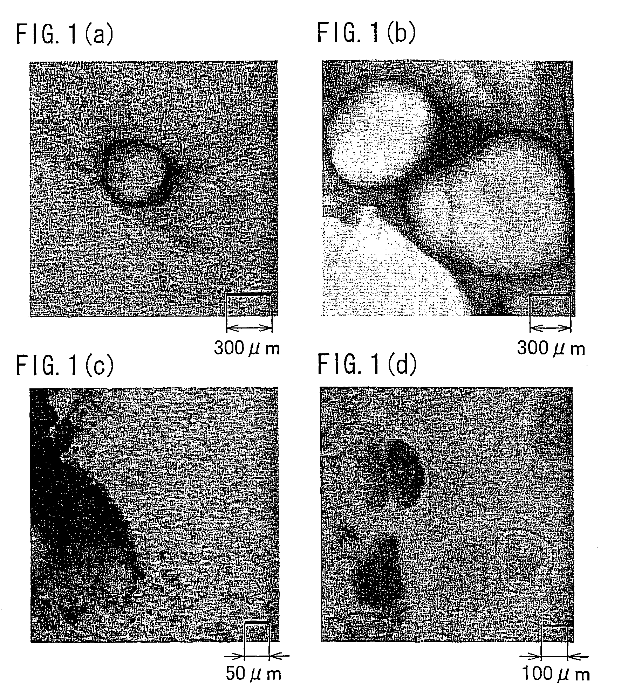

By a two-to-three-week incubation of the PB cells, the entire ES cell colonies grew into differentiated cells without pigmentation. Figures 1(a) to 1(d) show results of observation of the cells through a phase-contrast microscope. Figure 1(a) shows a result of observation of relatively small lentoids 30 days after the start of induction. Figure 1(b) shows a result of observation of large lentoids 53 days after the start of induction. As shown in Figures 1(a) and 1(b), the cultured cells finally accumulated and formed a transparent body that changed its size. A bar at the lower right of each figure indicates the scale bar.

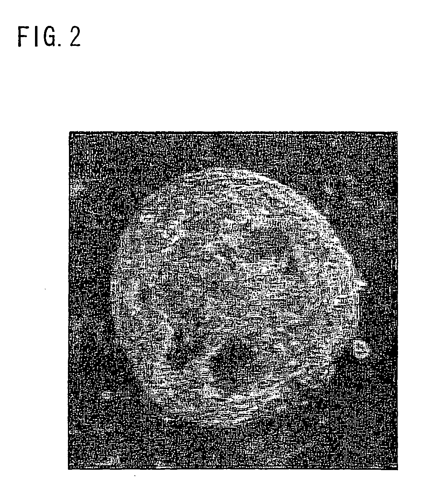

Figure 2 shows the result of immunostaining of induced cells. In Figure 2, the white regions indicate regions stained by immunostaining. From the result, it was confirmed that the induced cells contained anti-alpha-crystallin A antibodies, showing that the induced cells expressed alpha-crystallin A. As a result, the induced cells were characterized as lentoids.

Further, it was confirmed that some colonies formed retinal pigment epithelial cells (see Figure 1(c)). Most of the retinal pigment epithelial cells were generated in colonies independent from colonies containing lentoids. In some other colonies, lentoids and retinal pigment epithelial cells were observed together as they were actually observed by the naked eye. (see Figure 1(d)).

The lentoids were first observed 14 to 16 days after the start of induction. The proportion of colonies containing lentoids started to gradually increase 20 days after the start of induction. The number of lentoids reached a peak within 40 days from the start of induction.

### (2) Result of Analysis by Western-Blotting

The expression of alpha-crystallin A by the lentoids was further investigated by western-blotting analysis. Specifically, proteins of lentoids were transferred onto a PVDF film 40 days after the start of induction, and the proteins were electrophorased using anti-alpha-crystallin A antibody or anti-Pax 6 antibody as a probe.

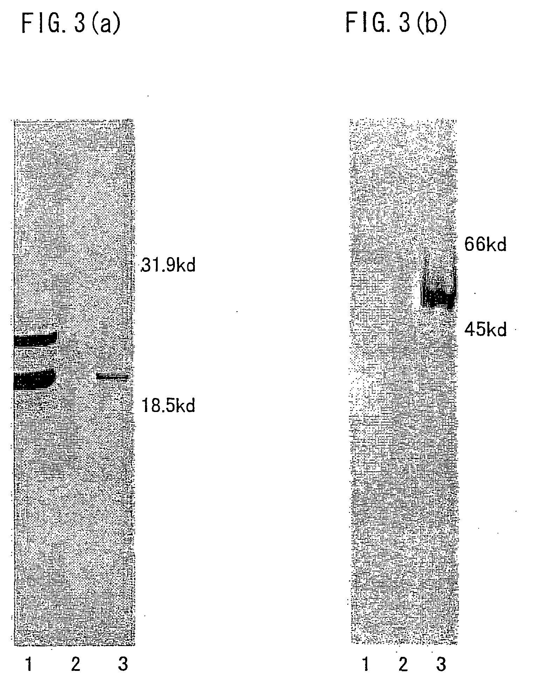

The results are shown in Figures 3(a) and 3(b). Figure 3(a) shows the result of western-blotting analysis in which expression of the alpha-crystallin A was examined. Lane 1 indicates positive control (lens protein of rat). Lane 2 indicates negative control (rat neural stem cell). Lane 3 indicates the total amount of lentoid protein according to the SDIA method. Figure 3(b) shows the result of western-blotting analysis in which expression of Pax6 was examined. In the figure, lane 1 indicates protein of the PA6 cells, lane 2 indicates the total amount of protein of undifferentiated ES cells, and lane 3 indicates the total amount of protein of lentoid cells according to the SDIA method.

As shown in Figure 3(a), the rabbit anti-alpha-crystallin-A polyclonal antibody detected 22 kDa protein. A single-band that indicates crystalline-alpha-protein was detected in a solution of ES cells that had been induced to differentiate by the SDIA method in lane 3. However, the single-band was not detected in the negative control (rat neural stem cell) in lane 2. Two bands (22 kDa and 25 kDa) were detected in the positive control (lens protein of rat) in Lane 1. It is believed that the 25 kDa band was A^{ins}, which is expressed in rat alpha-crystallin.

With regard to the expression of Pax6 indicated in Figure 3 (b), the rabbit anti-Pax6 antibody detected 48 kDa Pax-protein. It was confirmed that the Pax6 was expressed in the synthesized lentoids (lane 3), but there was no expression in the PA6 feeder-cells (lane 1) or undifferentiated ES cells (lane 2).

As reported in Furuta Y., Hogan BL., BMP4 is essential for lens induction in the mouse embryo, Genes Dev, 1998, Vol. 12, pp. 3764-3775, Pax6 is essential for the formation of lenses. The expression of Pax6 involving interaction between the optic vesicle and surface ectoderm induces formation of lens placode and invagination. Lack of Pax6 in a precursor of the lens causes defects in the formation of lenses.

In this experiment, it was confirmed that differentiation of ES cells induced expression of Pax6 in lentoids. This suggests that Pax6 is a key functional factor for the formation of lentoids from the ES cells and for normal development. In addition, the results prove that the lens can be actually formed by the differentiation of ES cells induced by the SDIA method.

### (3) With Regard to Influence of Exogenous FGF-2 in the Induction of Lentoids

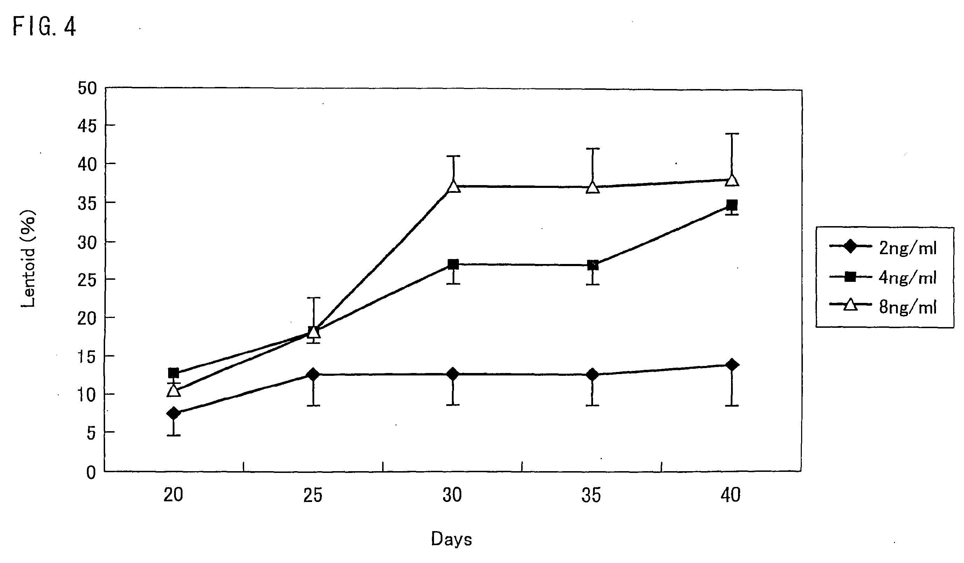

The influence of FGF-2 concentration in the induction of lentoids from primate ES cells was also evaluated. Figure 4 shows the result of evaluation. Figure 4 is a graph showing a relationship between the elapsed time from the start of induction and percent induction of lentoids, at different FGF-2 concentrations of 2 ng/ml, 4 ng/ml, and 8 ng/ml in the medium for maintaining undifferentiated ES cells. In Figure 4, the FGF-2 concentrations of 2 ng/ml, 4 ng/ml, and 6 ng/ml are indicated by "◆", "■", and "△", respectively.

As shown in Figure 4, no significant difference due to the difference of concentrations was observed in the first 20 days after the start of induction. Thirty days after the start of induction, the percentage of colonies containing lentoids started to show dose-dependent increase with increase in FGF-2 concentration. The inventors also attempted to add FGF-2 to ES differentiation medium. However, the PA6 cells grew so fast that differentiating ES cells could not be maintained (data not indicated).

Several research works reports that FGF plays an important role in differentiation and development of lenses. For example in mammals, it has been confirmed that a fibroblast growth factor (FGF) facilitates differentiation of lens fibrae (Chamberlain CG., McAvoy JW., Evidence that fibroblast growth factor promotes lens fiber differentiation, Curr Eye Res, 1987, Vol. 6, pp. 1165-1169). FGF-1 and FGF-2 are expressed in the neural retina and lens cells of mice during the developmental stage. A FGFR (FGF receptor)-1 and FGFR (FGF receptor)-2 are also expressed in the lens cells.

In the present experiment, it was found that increase in the concentration of FGF-2 in the medium maintaining the undifferentiated ES cells influenced differentiation of lentoids. In other words, it was confirmed that the induction of lentoid differentiation could be facilitated when the FGF-2 concentration in the maintenance medium was higher than 2 g/ml. This finding suggests the possibility of upregulating the receptors of the differentiation factors in the SDIA medium in order to cause the undifferentiated ES cells to effectively respond to the differentiation factors.

### (4) With Regard to Influence of Colony Density in the Induction of Lentoids

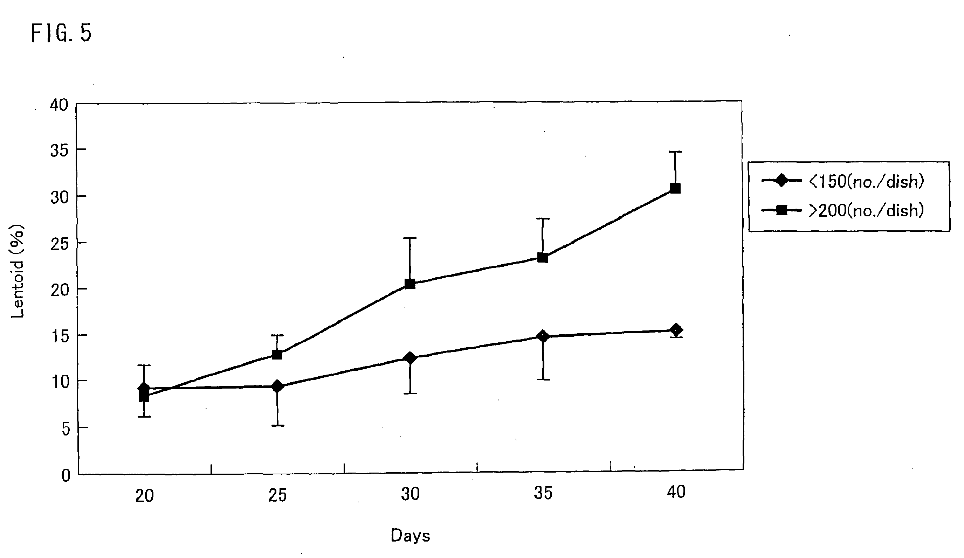

Figure 5 shows a relationship between ES cell-density and induction of lentoids concerning ES cells implanted on the PA6 feeder-cells. Figure 5 is a graph showing a relationship between the elapsed time from the start of induction and the percentage (%) of induced lentoids, at high ES cell density (no less than 200 colonies /10 cm culture dish: indicated with ■ in Figure 5) and low ES cell density (no less than 150 colonies /culture dish with inner diameter is 10 cm: indicated with ◆ in Figure 5).

As shown in Figure 5, the percentage of colonies containing lentoids proportionally increased with respect to the density of ES colonies added at the start of incubation.

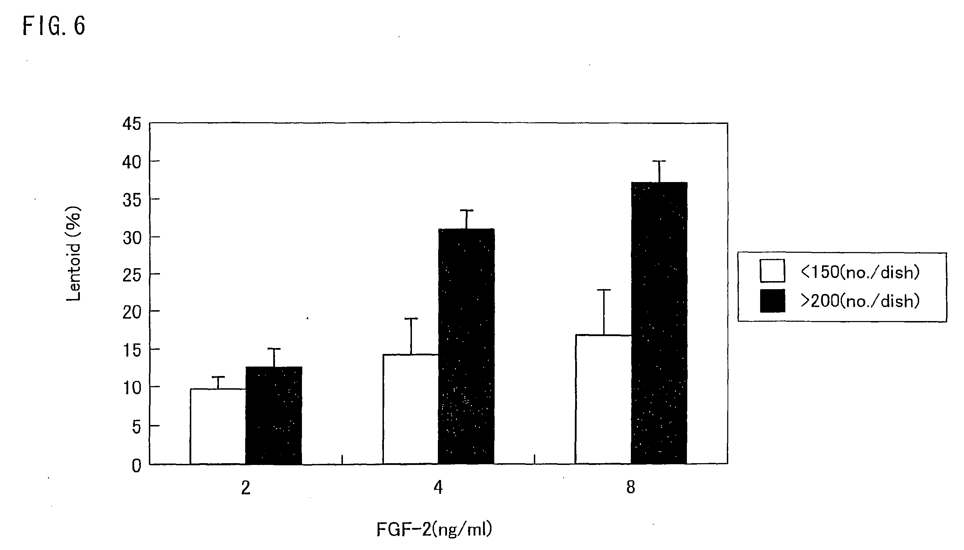

Figure 6 shows the influence of colony density at different FGF-2 concentrations thirty days after the start of induction. In the graph of Figure 6, percent induction of lentoids for the FGF-2 concentrations 2 ng/ml, 4 ng/ml, and 8 ng/ml is shown in this order from the left. In addition, at each FGF-2 concentration, the blank histogram indicates low colony density (no less than 150 colonies/culture dish with inner diameter of 10 cm), and the solid histogram indicates high colony density (no less than 200 colonies/10 cm-culture dish).

As shown in Figure 6, thirty days after the induction, the number of lentoids induced by the differentiation of ES cells was greater in the culture solution in which the ES cells were implanted in the PA6 cells at high density (no less than 200 colonies/10 cm-culture dish) than in the culture solution in which the ES cells were implanted at low density (no less than 150 colonies/10 cm-culture dish). Further, it was found that the number of lentoid cells linearly increased with increase in the density of ES cells that had been implanted prior to induction. The same result was observed forty days after the start of induction. However, no significant difference was observed twenty days after the start of induction.

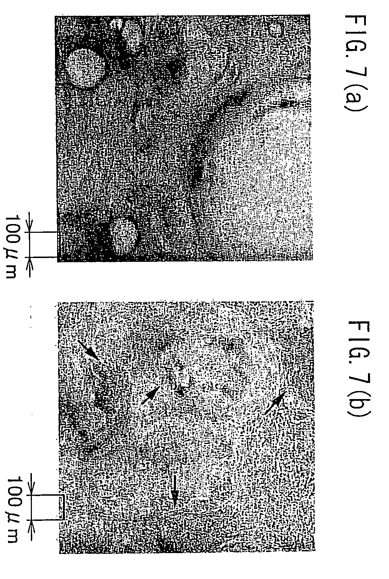

Figure 7(a) shows a result of microscopy on lentoids cultured at high colony density. It can be seen from the Figure that various types of lentoids were formed in several colonies. The scale-bar at the lower right indicates 100 µm. Figure 7(b) shows a result of microscopy on retinal pigmented epithelial cells cultured at high colony density. It can be seen from the Figure that the retinal pigmented epithelial cells, as pointed out by arrows, were formed in several colonies. The scale-bar at the lower right indicates 100 µm. In this manner, induction of retinal pigmented epithelial cells was enhanced in a culture solution with high colony density.

The above results suggest that the density of implanted colony might affect the level of lentoid induction. Intercellular contact is important for formation of lentoids in the lens epithelial cells of mice. Further, transdifferentiation of the neural retina of newborn chicks into lenses occurs under crowded conditions involving a number of stages. In this manner, formation of lentoids tends to occur in crowded conditions, and therefore colony density can be an important differentiation factor. Colony density may affect the level of differentiation of retinal pigmented epithelial cells. These data suggest that the SDIA treatment can facilitate formation of ocular cells under crowded colony conditions.

As a summary of the present experiment, it was confirmed that lentoids could be stably reproduced from the ES cells of cynomolgus monkey when the SDIA method was performed under increased FGF-2 concentrations in the medium maintaining the ES cells, and under increased colony density of the ES cells implanted on PA6.

It will be obvious that the invention thus described may take many variations. Such variations are not to be regarded as a departure from the spirit and scope of the invention, as all such variations would be obvious to one skilled in the art, and are hence intended to be included within the scope of the following claims.

### INDUSTRIAL APPLICABILITY

As described above, a method of producing a lens cell according to the present invention allows a lens cell to be stably produced from ES cells, and therefore provides lens cells that can be used as biomaterials for transplant, and lens fiber tissues formed of such lens cells. Because the lens cells produced by the above method constitute a group of homogeneous cells reproduced from the ES cells, the lens cells can be effectively utilized for development of biomaterials for transplant in regenerative treatment.

The present invention is the first case of successful induction of differentiation of ES cells of humans and other primates into lenses. The present invention not only has academic significance contributing to finding mechanisms relating to differentiation of ES cells but also may be utilized for research and development of therapeutic drugs for cataracts, contributing to development in pharmaceutical field. Therefore, the present invention is highly useful.

## Claims

1. A method of producing a lens cell comprising:
an ES cell maintenance step of maintaining an ES cell by using a medium containing a fibroblast growth factor FGF-2 at a concentration of 2 ng/ml to 50 ng/ml; and
a differentiation inducing step, carried out after the ES cell maintenance step, of inducing differentiation of the ES cell into a lens cell by implanting and culturing the ES cell on a mouse-skull-cell PA6 at a cell density of 2 colonies/cm² to 6.5 colonies/cm².

2. A method of producing a lens cell as set forth in Claim 1, further comprising a washing step, carried out between the ES cell maintenance step and the differentiation inducing step, of washing the maintained ES cell once with an ES differentiation medium.

3. A method of producing a lens cell as set forth in Claim 2, wherein the differentiation inducing medium used for inducing differentiation of the ES cell into a lens cell is used as the ES differentiation medium.

4. A method of producing a lens cell as set forth in any one of Claims 1 to 3, wherein, in the ES cell maintenance step, the medium contains the fibroblast growth factor FGF-2 at a concentration of 4 ng/ml to 50 ng/ml.

5. A method of producing a lens cell as set forth in any one of Claims 1 to 4, wherein, in the differentiation inducing step, the ES cell is implanted on the PA6 cell at a cell density of 2.5 colonies/cm² to 4.0 colonies/cm².

6. A method of producing a lens cell as set forth in any one of Claims 1 to 5, wherein the ES cell is derived from primates.

7. A method of producing a lens cell as set forth in Claim 6, wherein the ES cell is derived from cynomolgus monkey.

8. A lens cell obtained by a method of producing a lens cell set forth in any one of Claims 1 to 7.
